# EUROPEAN PATENT APPLICATION

(11) **EP 0 979 653 A1**
(43) Date of publication of application: **16.02.2000**
(21) Application number: 97944104.5
(22) Date of filing: 09.10.1997
(51) Int. Cl.: A61K 31/565

(54) **REMEDY FOR CHRONIC FATIGUE SYNDROME**

(30) Priority: 14.10.1996 JP 29341396
(71) Applicant: KANEBO, LTD., Tokyo 131 (JP)
(72) Inventor: KURATSUNE, Hirohiko, Toyonaka-shi, Osaka 560 (JP); KITANI, Teruo, Suita-shi, Osaka 565 (JP); SAWADA, Masumi, Osaka-shi, Osaka 545 (JP)
(74) Representative: VOSSIUS & PARTNER
(86) International application number: JP9703658
(87) International publication number: WO9816543

(57) **Abstract**

To provide a remedy for CFS, comprising dehydroepiandrosterone sulfate (DHAS) or a pharmaceutically acceptable salt thereof as an active ingredient.

By administering DHAS or a pharmaceutically acceptable salt thereof, as an active ingredient of the remedy for CFS of the present invention, to a patient with CFS, DHAS in plasma reduced in the patient with CFS is supplemented thereby to recover depressed immunity, whereby chronic lassitude, weakness and psychoneurotic symptom can be remitted.

## Description

### Technical Field

The present invention provides a novel remedy for chronic fatigue syndrome. More particularly, the present invention relates to a remedy for chronic fatigue syndrome, comprising dehydroepiandrosterone sulfate or a pharmaceutically acceptable salt thereof as an active ingredient.

### Background Art

In Europe and America, an intense interest has rapidly been shown towards disease concept of so-called chronic fatigue syndrome (hereinafter referred to as CFS) wherein its patient complains severe chronic lassitude enough to interfere with everyday life, recently. However, etiology of CFS has never been clarified, although an intensive study has been made from a lot of points of view, for example, various abnormalities in immunity including viral infectious disease, abnormality in energy metabolism, psychological etiology, etc. Under these circumstances, it is also considered that CFS is brought into existence under a complex mutual relation of two-sidedness, that is, fatigue as a mental/sensual side view and a state of fatigue which can be measured physiologically and biochemically in view of metabolism (Teruo KITANI, Concept of CFS, Clinical Chemistry, Vol. 29, No. 6, pages 663-668, 1993).

Accordingly, in the treatment of CFS, a trial of administering a Chinese herbal remedy (e.g. Hotyuuekkitoo, Juzentaiho-To, Ninzin-yoei-To, Rikkunsi-To, etc.), an anti-inflammatory agent, an antidepressant and an adjuvant and a trial of administering a large amount of vitamin C have been made as a symptomatic therapy, but an essential therapy have still to be found.

DHAS or a pharmaceutically acceptable salt thereof has an action of maturing the cervical canal at the end stage of gestation and an action of enhancing sensitivity of the uterine muscle to oxytocin (JP-B-55-27884) and a sodium salt thereof has widely been used as an agent for maturation of the cervical canal. It has also been known that DHAS or a pharmaceutically acceptable salt thereof is effective for treatment of demantia (JP-A-62-99328), prevention and treatment of hyperlipidemia (JP-A-64-40428), treatment of osteoporosis (JP-A-3-209328) and treatment of ulcer (JP-A-4-355983).

However, it has never been reported that DHAS or a pharmaceutically acceptable salt thereof is effective for treatment of CFS.

### Disclosure of the Invention

An object of the present invention is to provide a novel remedy for chronic fatigue syndrome.

As a result of an intensive study, the present inventors have noticed that the concentration of DHAS in plasma of a patient with CFS is lower than that of a healthy person (see Reference Example 1 described hereinafter) and administered DHAS or a pharmaceutically acceptable salt thereof to the patient with CFS. As a result, they have found that DHAS concentration in plasma increased thereby to recover depressed immunity, whereby chronic lassitude, weakness and psychoneurotic symptom can be remitted, that is, DHAS or a pharmaceutically acceptable salt thereof is useful for treatment of chronic fatigue syndrome, thus completing the present invention.

### Best Mode for carrying out the Invention

The pharmaceutically acceptable salt of DHAS used in the present invention includes, for example, salts of alkaline metals such as sodium, potassium, etc., hydrates thereof, or salts of organic amines such as arginine, ethanolamine, etc. Among them, a sodium salt or a hydrate thereof is preferred.

Usually, the remedy for chronic fatigue syndrome of the present invention may be orally or parentally administered to a human.

Examples of pharmaceutical preparation forms for oral administration include tablets, granules, fine granules, powders and the like, and these pharmaceutical preparations may be produced by appropriately mixing DHAS or a pharmaceutically acceptable salt thereof with conventional pharmaceutical additives such as lactose, corn starch, crystalline cellulose, magnesium stearate, hydroxypropylcellulose, talc and the like, using conventional methods.

Examples of pharmaceutical preparation forms for parenteral administration includes injections, suppositories, pessaries and the like. For example, injections are produced by appropriately dissolving DHAS or a pharmaceutically acceptable salt thereof, as a freeze-dried pharmaceutical preparation to be dissolved when using, and stabilizers (e.g. neutral or basic amino acids such as glycine, alanine, leucine, arginine, histidine, etc.) in purified water, using conventional methods. For example, suppositories and pessaries may be produced by dispersing DHAS or a pharmaceutically acceptable salt thereof, stabilizers and absorption accelerators (e.g. neutral or basic amino acids such as glycine, alanine, leucine, arginine, histidine, etc., or hydroxycarboxylic acids such as citric acid, L-tartaric acid, etc.) in a molten base for suppository such as hard fat having a hydroxyl value of not more than 50, filling the dispersion in a mold, and cooling it.

The dose of the remedy for chronic fatigue syndrome varies depending on the condition of the patient, administration route, age, sex or the like. Usually, the remedy as DHAS is administered to an adult patient at a time or 2 to 3 times per day with a dairy dose of 0.5 to 100 mg/kg.

By administering DHAS or a pharmaceutically acceptable salt thereof, as an active ingredient of the remedy for CFS of the present invention, to a patient with CFS, DHAS in plasma reduced in the patient with CFS is supplemented thereby to recover depressed immunity, whereby chronic lassitude, weakness and psychoneurotic symptom can be remitted, as shown in the following Test Examples (see Test Example 1 below).

The following Test Examples illustrate the effect of the present invention in detail.

### [Test Examples]

### Test Example 1

### (1) Test compound

Sodium salt of DHAS

### (2) Test method

An injection containing 100 mg of a test compound was intravenously administered to two patients with CFS (A, B), continuously, for 14 days. The measurement of DHAS concentration in plasma was conducted before administration and 13 hours after administration with respect to the first and second days, while the measurement was conducted before administration with regard to the third, fifth, eighth, eleventh and fourteenth days. On the fourteenth day from the day on which first administration was conducted, the patients were questioned about lassitude, weakness and psychoneurotic symptom.

With regard to the patient B, a natural killer (NK) activity was measured after administration on the first day and before administration on the fifteenth day. The measurement of the NK activity was conducted in accordance with the method described in Later Biological Experimental Course, No. 5, Process for Study of Immunobiochemistry (Incorporation of The Japanese Biochemistry Society ed., published by Tokyo Kagaku Dojin Co.), page 186. That is, the NK activity was measured by using, as an indication, a cytotoxic activity determined by using ⁵¹Cr-labelled K562 as a target cell. Incidentally, a ratio of effector cell to target cell (E/T ratio) was set at 10:1 or 20:1.

### (3) Test results

The measurement results of the concentration of DHAS in plasma (ng/ml) of the patients with CFS (A, B) are shown in Table 1. The NK activity of the patient B before and after administration is shown in Table 2. Furthermore, the results of the degree of improvement of the patients A, B on the fourteenth day from the day on which first administration was conducted are shown in Table 3.

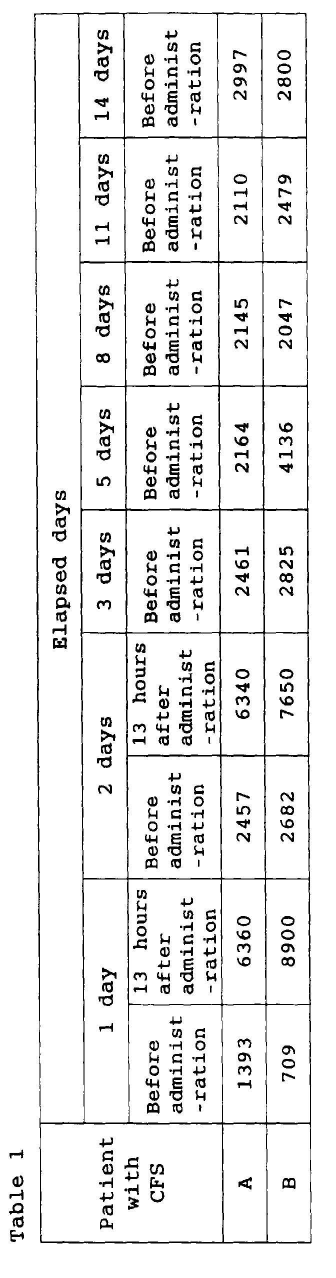

As is apparent from Table 1, the concentration of DHAS in plasma was increased by administering the remedy for chronic fatigue syndrome of the present invention to a patient with CFS.

**Table 2**

| E/T ratio | NK activity (%) | |
|---|---|---|
| | Before administration on the first day | After administration on the fourteenth day |
| 10:1 | 7.2 | 12.1 |
| 20:1 | 13.7 | 18.5 |

As is apparent from Table 2, in any case where the E/T ratio is 10:1 or 20:1, the NK activity was increased by administering the remedy for chronic fatigue syndrome of the present invention to a patient with CFS, thereby to recover the immunity of the patient with CFS.

**Table 3**

| Patient with CFS | Degree of improvement on the fourteenth day | | |
|---|---|---|---|
| | Lassitude | Weakness | Psychoneurotic symptom |
| A | improved | Improved | Improved |
| B | improved | Improved | Improved |

As is apparent from Table 3, lassitude, weakness and psychoneurotic symptom of a patient with CFS were improved.

The following Example and Reference Examples further illustrate the present invention in detail.

### Example 1 (tablets)

### [Formula]

| Components | Amount (g) |
|---|---|
| Dihydrate of sodium salt of DHAS | 109.2 |
| Lactose | 64.8 |
| Corn starch | 31.6 |
| Hydroxypropylcellulose | 1.8 |
| Magnesium stearate | 0.6 |

### [Procedure]

After dihydrate of a sodium salt of DHAS, lactose and corn starch are admixed, the mixture was passed through a 60-mesh sieve. To this mixture is added an aqueous hydroxypropylcellulose solution, followed by kneading. The paste is granulated, dried, mixed with magnesium stearate and compressed to obtain tablets of 208 mg each, which contain a sodium salt (anhydrate) of DHAS in an amount of 100 mg per tablet.

### Example 2 (injections)

### [Formula]

| Components | Amount (g) |
|---|---|
| Dihydrate of sodium salt of DHAS | 43.7 |
| Glycine | 40.0 |

### [Procedure]

Glycine is dissolved by adding purified water and to this is added dihydrate of a sodium salt of DHAS with heating, followed by dissolving to make 2000 ml as the total amount. After this solution is sterilized, 5 ml of each solution is filled in a bial for injection and freeze-dried to obtain injections to be dissolved when using, which contain a sodium salt (anhydrate) of DHAS in an amount of 100 mg per bial.

### Example 3 (pessaries)

### [Formula]

| Components | Amount (g) |
|---|---|
| Dihydrate of sodium salt of DHAS | 43.7 |
| Glycine | 40.0 |
| Hard fat | 266.3 |

### [Procedure]

Hard fat [Witep Sol™ H-15, manufactured by Huls AG Co.] is charged in a beaker made of stainless steel and then molten by heating to 40-50°C. To this is added dihydrate of a sodium salt of DHAS and glycine, followed by stirring and further uniform mixing. This mixture (1.6 g each) is filled in a spindle-shaped mold with maintaining at 37-55°C, and then cooled to obtain pessaries containing a sodium salt (anhydrate) of DHAS in an amount of 200 mg per one pessary.

### Reference Example 1 (Comparison in concentration of DHAS in plasma between patient with CFS and healthy person)

The concentration of DHAS in plasma of patients with CFS (male: 4, female: 21) and that of healthy persons (male: 36, female: 35) were measured. The results are shown in Table 4.

**Table 4**

| Sex | Concentration of DHAS in plasma (ng/ml, average ± standard deviation) | |
|---|---|---|
| | Patients with CFS | Healthy persons |
| Male | 1858 ± 894 | 3441 ± 1059 |
| Female | 1597 ± 618 | 2542 ± 963 |

## Claims

1. A remedy for chronic fatigue syndrome, comprising dehydroepiandrosterone sulfate or a pharmaceutically acceptable salt thereof as an active ingredient.

2. A remedy for chronic fatigue syndrome, comprising a sodium salt of dehydroepiandrosterone as an active ingredient.
